# FASCICULE DE BREVET EUROPEEN

(11) **EP 0 550 610 B1**
(45) Date de publication et mention de la délivrance du brevet: **23.08.1995**
(21) Numéro de dépôt: 91917601.6
(22) Date de dépôt: 26.09.1991
(51) Int. Cl.: C12Q 1/68, C12Q 1/04, C12P 19/34, C07H 21/04

(54) **PROCEDES, OLIGONUCLEOTIDES AMORCES ET OLIGONUCLEOTIDES SONDES POUR LA DETECTION DE BACTERIES PATHOGENES DE LA CAVITE BUCCALE**
VERFAHREN, OLIGONUKLEOTIDPRIMERS UND OLIGONUKLEOTIDSONDEN ZUM NACHWEIS VON PATHOGENEN BAKTERIEN DER MUNDHÖHLE
PROCESSES, OLIGONUCLEOTIDE PRIMERS AND OLIGONUCLEOTIDE PROBES FOR THE DETECTION OF PATHOGENIC BACTERIA IN THE BUCCAL CAVITY

(30) Priorité: 26.09.1990 FR 9011849
(43) Date de publication de la demande: 14.07.1993
(73) Titulaire: GENSET, 75011 Paris (FR)
(72) Inventeur: D'AURIOL, Luc, F-75010 Paris (FR); MONTPLAISIR, Nicole, F-75006 Paris (FR)
(74) Mandataire: Warcoin, Jacques
(86) Numéro de dépôt international: FR9100751
(87) Numéro de publication internationale: WO9205279

(56) Documents cités:
- EP-A- 199 439
- EP-A- 364 255
- WO-A-89/06704
- CHEMICAL ABSTRACTS, vol. 109, no. 9, août 1988, Columbus, OH (US); U. GOEBEL et al., p. 347, no. 69683m#

## Description

L'invention décrite ici concerne une procédure complète de détection de bactéries pathogènes dans la cavité buccale humaine, et plus particulièrement celles associées aux parodontopathies.

Le parodonte est constitué des tissus de soutien de la dent. Le terme de maladie parodontale destructrice englobe un ensemble de formes cliniques de gingivites et parodontites, maladies inflammatoires ayant pour facteur étiologique des micro-organismes. Ces maladies parodontales se traduisent par une inflammation gingivale, une lyse osseuse, la formation de poche parodontale par migration apicale de la jonction gingivo-dentaire et aboutissent à terme à la perte de la dent après destruction des tissus de soutien de cette dent.

Les études épidémiologiques montrent que l'incidence et la sévérité des parodontopathies sont variables suivant les pays et les groupes d'âge, mais des études récentes portant sur 147 millions d'Américains de 19 ans et plus évaluent a environ 20% les dents perdues à cause de la maladie parodontale.

L'incidence la plus importante d'extraction dentaire pour cause de maladie parodontale survient entre 20 et 50 ans. Après 35 ans, la majorité des dents perdues le sont pour cause de maladie parodontale plutôt que pour cause de carie dentaire. D'autre part, l'incidence de la carie dentaire est actuellement en nette régression dans les pays développés. De ce fait, davantage de gens gardent leurs dents au cours de leur vie, et la proportion de gens âgés augmentant, il est raisonnable de prévoir que la fréquence de la maladie parodontale augmentera dans le futur.

De nombreuses études ont étayé le fait que les micro-organismes de la plaque dentaire sont les agents étiologiques de la quasi-totalité des parodontopathies. On sait actuellement que des groupes spécifiques de bactéries sont associes avec des formes cliniques précises : 300 espèces bactériennes sont recensées dans la cavité buccale humaine, mais seulement 15 à 20 d'entre elles sont associées à la maladie parodontale. Il est donc probable que la plupart des micro-organismes de la plaque dentaire représentent des bactéries opportunistes dépourvues de pouvoir pathogène réel, un petit nombre seulement d'entre elles étant réellement impliqué dans l'étiopathogénie des parodontites.

Parmi celles-ci, deux ont été particulièrement reconnues comme les espèces majeures dans la plupart des maladies parodontales très évolutives : ce sont Bacteroides gingivalis et Actinobacillus actinomycetemcomitans.

On sait actuellement que la maladie parodontale n'est pas un processus lent, continu progressant de la gingivite à la parodontie terminale et à la perte des dents. Il existe au contraire des épisodes courts d'activité alternant avec de longues périodes de rémission : certains sites en particulier présentent des périodes d'activité de durée et d'amplitude variables aboutissant à des pertes d'ancrage et à des destructions osseuses importantes. Ces périodes d'activité ne peuvent, à l'heure actuelle, être reconnues qu'à posteriori, par la constatation d'une aggravation de la profondeur de poche par comparaison avec des mesures antérieures. Il n'existe, en effet, aucun critère diagnostique capable de détecter un site en phase d'activité. Cependant, les corrélations établies entre la présence de certains organismes spécifiques et l'aggravation des lésions permet de penser qu'un diagnostic microbiologique est essentiel pour : 1- évaluer les agents pathogènes, 2- déterminer les individus et les sites à risques, 3-déterminer le choix des méthodes thérapeutiques, 4- évaluer les effets du traitement, 5- détecter de façon précoce les sites réfractaires ou récidivants, 6- préciser les intervalles entre les visites.

Les techniques classiques permettant de détecter et de caractériser ces bactéries pathogènes font appel à des cultures bactériologiques qui, de par la nature particulière des anaérobies ou micro aérophiles concernées, ne sont réalisables que dans des environnements spécialisés. Mais le problème majeur de ces analyses est celui du prélèvement et de la conservation des bactéries vivantes : à partir d'un prélèvement effectué au cabinet du praticien, il est particulièrement délicat de conserver et de transporter dans de bonnes conditions des bactéries anaérobies pouvant être détruites par suite d'une simple exposition à l'oxygène de l'air. Des systèmes de confinement spéciaux et des procédures particulières doivent être utilisées lors de ces prélèvements. Ces lourds problèmes de manipulation limitent considérablement les possibilités d'utilisation de ces techniques microbiologiques et, en tout état de cause, condamnent leur usage en routine.

D'autres méthodes de diagnostic faisant appel à des reactions immunologiques et utilisant des anticorps poly ou monoclonaux reconnaissant certaines de ces bactéries ont été proposes et pourraient constituer des approches intéressantes.

Alternativement aux méthodes microbioiogiques et immunologiques. il est possible, pour rechercher la présence de tel ou tel micro-organisme. d'utiliser des sondes nucléotidiques spécifiques. Ces techniques ont déjà été appliquées avec succès aux pathogènes de la cavité buccale et plusieurs sondes ont déjà été décrites par plusieurs laboratoires. Les tests consistent alors en une hybridation directe entre l'ADN provenant d'un prélèvement et une sonde marquée caractéristique d'un des micro-organismes concernés.

Par exemple, le document "Dtsch. Zahnärztl. Z., 43, 661-664 (1988)" décrit des sondes oligonucléotidiques complémentaires de l'ARN 16S d'une souche des espèces Actinobacillus actinomycetemcomitans, Bacteroides asaccharolyticus, B. gingivalis et B. intermedius ainsi que leur utilisation par une technique d'hybridation sur filtre.

Si ces tests donnent des résultats satisfaisants sur des échantillons contenant une concentration élevée de bactéries, ils rencontrent les limites techniques inhérentes à l'activité spécifique de la sonde marquée et au nombre de molécules cibles présentes dans l'échantillon à analyser, et ne permettent pas de détecter de faibles quantités de bactéries.

Or, il semble bien que le plus souvent l'évolutivité à long terme d'une poche de parodontie soit directement liée à la présence résiduelle de micro-organismes pathogènes dont le traitement ne serait pas venu à bout. Les traitements mécaniques ou chimiothérapeutiques utilisés dans les parodonties visent en effet à éradiquer totalement ces bactéries afin d'arrêter le processus infectieux responsable des lésions du parodonte. Il est donc fondamental de pouvoir évaluer avec précision si cet objectif a été atteint et si des bactéries restent présentes dans les poches. Qu'un foyer de bactéries demeure, et la lésion peut poursuivre son évolution à bas bruit. Un e analyse par simple hybridation directe peut ne pas être en mesure de déceler de faibles populations bactériennes. Préalablement à cette hybridation, une étape d'amplification de la cible s'avère donc nécessaire afin de se trouver dans des zones de sensibilité optimale.

### OBJET ET PRINCIPE DE L'INVENTION

L'objet de la présente invention est de décrire les procédures permettant une amplification et une détection optimale de l'ADN de ces pathogènes.

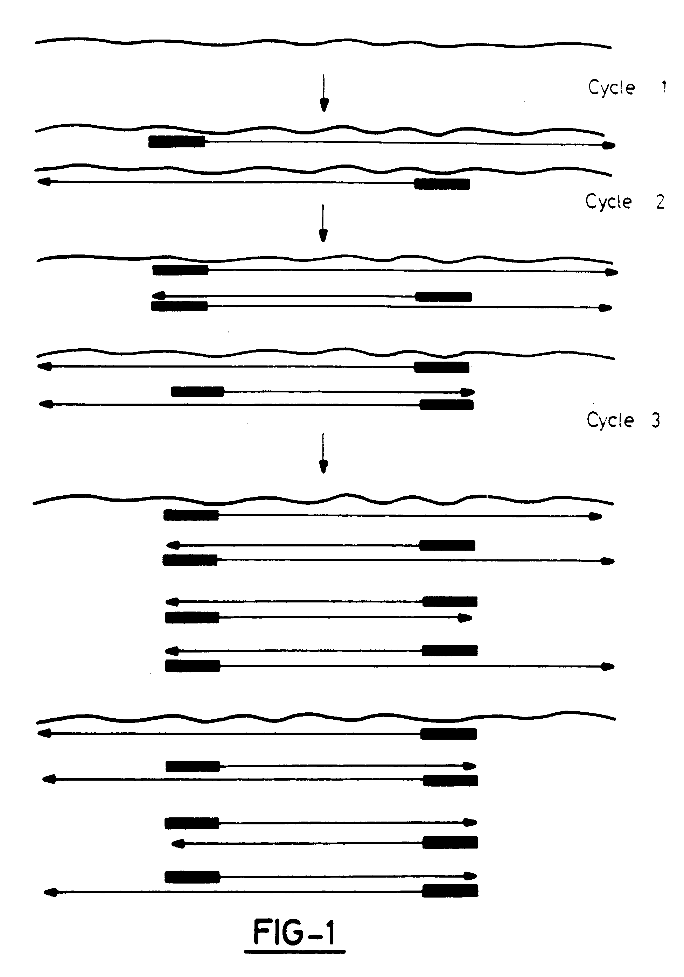

Cette invention fait appel, pour l'étape d'amplification des séquences cibles, à l'utilisation de la technique PCR (Polymerase Chain Reaction). Cette réaction est basée sur le principe de la réplication de l'ADN par duplication et sur l'utilisation d'amorces spécifiques, encadrant la séquence cible à amplifier, ces amorces étant utilisées comme point de démarrage pour des enzymes de réplication de l'ADN, en particulier pour des ADN polymérases thermostables utilisées pour ces réactions. Le schéma de principe de cette technique est rappelé figure 1.

L'amplification de la séquence cible procède par cycles successifs comportant une étape de dénaturation de l'ADN, puis une hybridation des amorces sur les séquences qui leur sont complémentaires, et enfin par l'élongation effectuée par l'ADN polymérase à partir de ces amorces. Après un tel cycle, une séquence cible présente en x exemplaire(s) dans l'échantillon à amplifier se retrouvera dupliquée, c'est-à-dire présente en 2x exemplaires. Après n cycles d'amplification, le nombre de séquences cibles sera de x.2ⁿ. Les procédures de PCR couramment utilisées font état de 20 ou 30 cycles d'amplification, ce qui correspond à des facteurs d'amplification de plusieurs dizaines de millions de fois.

L'utilisation d'une telle technique permet donc de diminuer considérablement le seuil de sensibilité pour la détection de microorganismes et, théoriquement, permet de le descendre jusqu'au niveau de la cellule unique.

Les techniques d'amplification fondées sur la PCR sont tellement puissantes qu'elles permettent même de visualiser directement, par des artifices de coloration, le fragment d'ADN produit par l'amplification. Cependant, au cours des cycles des reactions de polymérisation artéfactuelles peuvent produire des produits d'amplification ne correspondant pas toujours à la région d'ADN ciblée. Afin d'assurer une spécificité absolue dans la caractérisation des produits d'amplification, on procède, sur ces produits, a une hybridation avec une sonde nucléotidique reconnaissant spécifiquement une région d'ADN située entre les amorces utilisées dans l'amplification. Cette hybridation, effectuée sur des produits homogènes présents en concentration élevée, ne rencontre évidemment pas les mêmes problèmes de sensibilité qu'une hybridation conduite directement sur les prélèvements et permet, si on le souhaite, d'utiliser pour le marquage de la sonde toutes sortes de marquages froids à faible activité spécifique et bruits de fond faibles ou nuls.

De plus, les amorces et les sondes oligonucléotidiques utilisées pour ce système sont des fragments d'ADN synthétique fabriqués par synthèse chimique. L'utilisation d'ADN de synthèse est un facteur de garantie des séquences utilisées et permet une meilleure maîtrise des conditions expérimentales.

Ce système exploite donc deux niveaux de spécificité : d'une part, on utilise des amorces oligonucléotidiques spécifiques de la famille bactérienne et des conditions d'hybridation strictes et spécifiques et d'autre part, on repère le produit amplifié avec une sonde oligonucléotidique spécifique de la bactérie recherchée. Cette double réaction d'hybridation, sur trois séquences cibles non directement contigües, garantit la haute spécificité de cette approche.

C'est pourquoi la présente invention concerne un procédé pour la mise en évidence d'une bactérie Bacteroides gingivalis ou Actinobacillus actinomycetemcomitans de la cavité buccale, caractérisé en ce que:
- on prélève un échantillon dans la cavité buccale,
- après préparation de l'échantillon, on amplifie un ADN cible situé entre deux amorces de ladite bactérie, par le procédé PCR, mettant en oeuvre lesdites amorces, les amorces pour la détection de l'ADN cible de *Bacteroides gingivalis* ayant les séquences suivantes:
   Bg1 : 5'CTC TTG CTA TGA CAG CTT GT3'
   Bg2 : 5'AGT CAG TTC AGT TGT CAA TG3', et
   les amorces pour la détection de l'ADN cible de *Actinobacillus actinomycetemcomitans* ayant les séquences suivantes:
   Aa1 : 5'GGG TCT GGA GTA CTT TAG GG3'
   Aa2 : 5'CCC AGG CGG TCA GAT TTA3',
- après amplification, on hybride les produits d'amplification de l'ADN cible avec une sonde spécifique de l'ADN cible, et
- on révèle l'hybridation.

L'invention concerne également des amorces pour la mise en oeuvre de la méthode PCR de même que des sondes utilisables dans la méthode PCR, mais également dans les procédés d'hybridation classiques.

Plus particulièrement, il s'agit d'amorces pour la détection de l'ADN cible de Bacteroides gingivalis, caractérisées en ce qu'il s'agit des séquences suivantes :
Bg1 : 5'CTC TTG CTA TGA CAG CTT GT3'
Position 174 à 193 (Dickinson et al., J. Bact., 170, pp. 1658 - 1665)
Bg2 : 5'AGT CAG TTC AGT TGT CAA TG3'
Position 499 à 480 (Dickinson et al., J Bact., 170, pp. 1658 - 1665) ou d'une séquence similaire efficace utilisable pour l'amplification d'un ADN cible spécifique;
et/ou
d'amorces pour la détection de l'ADN cible de Actinobacillus actinomycetemcomitans, caractérisées en ce qu'il s'agit des séquencés suivantes :
Aa1 : 5'GGG TCT GGA GTA CTT TAG GG3'
Position 106 à 125 (Chuba et al., Jour. of Microbiol., 134,pp.1923-1930,1988)
Aa2 : 5'CCC AGG CGG TCA GAT TTA3'
Position 352 à 345 (Chuba et al.)
ou d'une séquence similaire efficace utilisable pour l'amplification d'un ADN cible spécifique.

Egalement, l'invention concerne :
- la sonde spécifique de l'ADN cible de Bacteroides gingivalis, caractérisée en ce qu'il s'agit de la sonde :
   Bg : GTT GAA GAC ATC AAA TGT AGT GCA GGC CAA
   ou une séquence équivalente efficace; et/ou
- la sonde spécifique de l'ADN cible de Actinobacillus actinomycetemcomitans, caractérisée en ce qu'il s'agit de la sonde
   AAC18 : CCC CAA ATC GGG ACC ACG
   ou une séquence équivalente efficace.

Enfin, l'invention concerne des trousses pour la mise en oeuvre dudit procédé.

Les terminologies "sondes" et "amorces" ("primer") sont connues dans cette technique et ne sont pas redéfinies.

On appelle ADN cible, l'ADN qui doit être amplifié par amplification d'un ADN spécifique d'une souche qui sera la cible de l'amplification.

Pour les amorces, celles-ci sont identifiées spécifiquement, mais il est possible, compte tenu de la connaissance des génomes bactériens d'utiliser des amorces voisines qui conduisent au même produit d'amplification de l'ADN cible, elles sont dites séquences similaires efficaces.

De même, une séquence équivalente efficace pour la sonde spécifique est une séquence qui permet d'identifier le même produit d'amplification.

### PROCEDURES UTILISEES

Les procédures décrites ici sont applicables à la recherche et à la caractérisation de toutes sortes de micro-organismes pouvant être présents dans la cavité buccale humaine et associés par exemple, soit à des caries soit à des parodonties.

Ces procédures ont cependant été mises au point en vue de la caractérisation spécifique de deux agents anaérobies, Actinobacillus actinomycetemcomitans et Bacteroides gingivalis, qui semblent être des contaminants majeurs des proches de parodontie en activité et en évolution. Ces deux cas seront donnes en exemple pour illustrer l'utilisation de cette technique.

### CHOIX ET PREPARATION DES AMORCES OLIGONUCLEOTIDIQUES

Les amorces oligonucléotidiques doivent répondre à plusieurs exigences :
- les amorces d'amplification doivent reconnaître tous les sous-types de la souche cible et encadrer une région hautement spécifique qui sert de cible à la sonde;
- les sondes doivent aussi reconnaître tous les sous-types et eux seuls.

Ceci pour respecter les exigences de spécificité et de sensibilité maxima. De plus, ces séquences doivent remplir certaines conditions exigées pour une amplification optimale, ne pas présenter de réactions d'hybridation intra- ou inter-moléculaire l'une par rapport à l'autre et avoir un rapport G+C/A+T satisfaisant.

Afin de rechercher de telles séquences spécifiques, de nombreux essais ont été réalisés sur des séquences connues de Bacteroides gingivalis et d'Actinobacillus actinomycetemcomitans.

Dans le cas de Bacteroides gingivalis nous nous sommes adressés à la séquence d'un gène codant pour une protéine fibrilaire caractéristique de Bacteroides gingivalis (Dickinson et al., J. Bact., 170, 4, pp 1658-1665, 1988). En analysant plusieurs couples d'amorces et différentes sondes, nous avons déterminé deux amorces appelées BG1 et BG2 et une sonde appelée BG. La spécificité de ce système a été vérifiée en effectuant des essais d'amplification et d'hybridation de ces produits sur les bactéries suivantes : B. gingivalis, B. melanogenus, B. corporis. B. oris, B. oralis, B. asaccnararolytus, B. ondodontalis, B. intermedius, B. denticola, B. loeschei. Dans les conditions définies ci-après seules les B. gingivalis donnent des résultats positifs.

Dans le cas d'Actinobacillus actinomycetemcomitans, on a fait appel pour sélectionner les séquences adéquates. à l'ARN ribosomal 165. Il est connu que l'ARN ribosomal comporte des régions qui sont hautement conservées de façon inter- et intra-spécifique et des régions spécifiques d'espèces. de genre, de type et sous-type.

Dans ce système deux amorces spécifiques ont été synthétisées correspondant à une séquencé conservée d'ARN ribosomal 165 (Chuba et al.. Journal of Microbiology, 134, pp 1923-1930, 1988). Les fragments amplifiés correspondant aux bactéries suivantes de la famille des Pasteurellaceae ont été séquencés :
- Actinobacillus lignieresi
- Hemophilus aphrophilus
- Hemophilus influenzae
- Actinobacillus equuli
- Actinobacillus actinomycetemcomitans 6330
- Actinobacillus actinomycetemcomitans 52106
- Actinobacillus hominis
- Actinobacillus multocida
- Actinobacillus actinomycetemcomitans 101032
- Actinobacillus actinomycetemcomitans 101033.

Plusieurs divergences majeures ont été trouvées par rapport aux séquences publiées. Ce travail nous a permis de déterminer une sonde spécifique du genre Actinobacillus actinomycetemcomitans qui ne reconnaît que cette bactérie à l'exclusion de toutes les autres citées ci-dessus.

Ces résultats ont été validés sur 20 sujets sains qui étaient tous négatifs pour Bacteroides gingivalis comme pour Actinobacillus actinomycetemcomitans.

En revanche, chez des patients atteints de diverses formes de parodontites, nous avons pu mettre en évidence la présence indépendante ou simultanée des deux bactéries.

### PROTOCOLES UTILISES

### I - TECHNIQUE DE PRELEVEMENT DES ECHANTILONS A TESTER

1) Elimination en douceur sur le site à prélever de la plaque supra-gingivale. Rinçage au spray.
2) Isolation à l'aide d'un rouleau de coton et séchage.
3) Insertion d'une pointe de papier absorbante pour endodontie, taille moyenne jusqu'au fond de la poche, en évitant de faire saigner, la pointe reste en place 15 secondes et est déposée dans un tube stérile.

### II - TRANSPORT

Les échantillons sont transportés en tube stérile à température ambiante.

### III - PREPARATION DES ECHANTILLONS AVANT LA MISE EN OEUVRE DE LA PROCEDURE D'AMPLIFICATION

Les pointes de prélèvement sont transvasées dans des tubes contenant 50 »l de tampon d'amplification et chauffés 10 min à 100°C.

### IV - AMPLIFICATION

Pour Bacteroides gingivalis les oligonucléotides employés sont :
Bg1 : 5'CTC TTG CTA TGA CAG CTT GT3'
Position 174 à 193 (Dickinson et al.)
Bg2 : 5'AGT CAG TTC AGT TGT CAA TG3'
Position 499 à 480 (Dickinson et al.)
Pour Actinobacillus actinomycetemcomitans
Aa1 : 5'GGG TCT GGA GTA CTT TAG GG3'
Position 106 à 125 (Chuba et al.)
Aa2 : 5'CCC AGG CGG TCA GAT TTA3'
Position 352 à 345 (Chuba et al.)
Les cycles d'amplification comprenaient :
- lère dénaturation 92°C 5 min
- lère hybridation 50°C 1 min
- lère élongation 72°C 1 min
puis 35 cycles
- dénaturation 92°C 1 min
- hybridation 50°C 1 min
- élongation 72°C 1 min
Pour rendre la mise en pratique de la détection de ces deux pathogènes plus rapide, nous réalisons les deux amplifications dans le même tube, le volume final étant de 100 »l. 250 ng de chaque oligonucléotide sont utilisés ainsi que 2,5 U de Taq polymérase.

### V - HYBRIDATION ET REVELATION

Les produits d'amplification précipités et resuspendus en 1SSC sont hybridés en tube à 60°C pendant une heure puis migrés en gel de polyacrylamide 6%.

Les deux sondes spécifiques respectivement de Aa : AaC18 et Bg : BG marquées au 32P, sont hybridées simultanément dans le même tube.
Sonde AaC18 : CCC CAA ATC GGG ACC ACG
Sonde Bg : GTT GAA GAC ATC AAA TGT AGT GCA GGC CAA
Une autoradiographie de 12 heures permet la révélation des résultats.

Tout autre système de marquage pourrait être utilisé.

## Revendications

1. Procédé pour la mise en évidence d'au moins une bactérie *Bacteroides gingivalis* ou *Actinobacillus actinomycetemcomitans* de la cavité buccale impliquée dans les parodontopathies, caractérisé en ce que:
- on prélève un échantillon dans la cavité buccale,
- après préparation de l'échantillon, on amplifie un ADN cible situé entre deux séquences amorces de ladite bactérie par le procédé PCR, mettant en oeuvre les dites amorces, les amorces pour la détection de l'ADN cible de *Bacteroides gingivalis* ayant les séquences suivantes:
Bg1 : 5'CTC TTG CTA TGA CAG CTT GT3'
Bg2 : 5'AGT CAG TTC AGT TGT CAA TG3', et
les amorces pour la détection de l'ADN cible de *Actinobacillus actinomycetemcomitans* ayant les séquences suivantes:
Aa1 : 5'GGG TCT GGA GTA CTT TAG GG3'
Aa2: 5'CCC AGG CGG TCA GAT TTA3',
- après amplification, on hybride les produits d'amplification de l'ADN cible avec une sonde spécifique de l'ADN cible, et
- on révèle l'hybridation.

2. Procédé selon la revendication 1, caractérisé en ce que, pour mettre en évidence plusieurs bactéries, on effectue l'amplification à l'aide des amorces compatibles aux dites bactéries dans le même récipient.

3. Amorces pour la détection de l'ADN cible de *Bacteroides gingivalis* ayant les séquences suivantes:
Bg1 : 5'CTC TTG CTA TGA CAG CTT GT3'
Bg2 : 5'AGT CAG TTC AGT TGT CAA TG3'.

4. Amorces pour la détection de l'ADN cible de *Actinobacillus actinomycetemcomitans* ayant les séquences suivantes:
Aa1 : 5'GGG TCT GGA GTA CTT TAG GG3'
Aa2 : 5'CCC AGG CGG TCA GAT TTA3'.

5. Sonde spécifique de l'ADN cible de *Bacteroides gingivalis* , caractérisée en ce qu'il s'agit de la sonde:
Bg: GTT GAA GAC ATC AAA TGT AGT GCA GGC CAA.

6. Sonde spécifique de l'ADN cible de *Actinobacillus actinomycetemcomitans* , caractérisée en ce qu'il s'agit de la sonde
AaC18: CCC CAA ATC GGG ACC ACG.

7. Trousse pour la mise en oeuvre du procédé selon l'une des revendications 1 ou 2.

8. Trousse selon la revendication 7, caractérisée en ce que les amorces et/ou les sondes sont celles des revendications 3 à 6.

## Claims

1. Process for the detection of at least one bacterium Bacteroides gingivalis or Actinobacillus actinomycetemcomitans of the buccal cavity which is involved in periodontopathies, characterized in that:
- a sample is collected from the buccal cavity,
- after preparation of the sample, a target DNA, situated between two primer sequences of the said bacterium, is amplified by the PCR process, using the said primers, the primers for the detection of the target DNA of Bacteroides gingivalis having the following sequences:
Bg1: 5'CTC TTG CTA TGA CAG CTT GT3'
Bg2: 5'AGT CAG TTC AGT TGT CAA TG3', and
the primers for the detection of the target DNA of Actinobacillus actinomycetemcomitans having the following sequences:
Aa1: 5'GGG TCT GGA GTA CTT TAG GG3'
Aa2: 5'CCC AGG CGG TCA GAT TTA3'.
- after amplification, the products of amplification of the target DNA are hybridized with a probe specific for the target DNA, and
- the hybridization is revealed.

2. Process according to Claim 1, characterized in that, to detect several bacteria, the amplification is carried out with the aid of primers compatible with the said bacteria, in the same container.

3. Primers for the detection of the target DNA of Bacteroides gingivalis having the following sequences:
Bg1 : 5'CTC TTG CTA TGA CAG CTT GT3'
Bg2 : 5'AGT CAG TTC AGT TGT CAA TG3'.

4. Primers for the detection of the target DNA of Actinobacillus actinomycetemcomitans having the following sequences:
Aa1: 5'GGG TCT GGA GTA CTT TAG GG3'
Aa2: 5'CCC AGG CGG TCA GAT TTA3'.

5. Probe specific for the target DNA of Bacteroides gingivalis, characterized in that it is the probe:
Bg: GTT GAA GAC ATC AAA TGT AGT GCA GGC CAA.

6. Probe specific for the target DNA of Actinobacillus actinomycetemcomitans, characterized in that it is the probe
AaC18: CCC CAA ATC GGG ACC ACG.

7. Kit for the implementation of the process according to either of Claims 1 and 2.

8. Kit according to Claim 7, characterized in that the primers and/or the probes are those of Claims 3 to 6.

## Patentansprüche

1. Verfahren zum Nachweis mindestens eines Bakteriums Bacteroides gingivalis oder Actinobacillus actinomycetemcomitans in der Mundhöhle, das an den Parodontopathien beteiligt ist, dadurch gekennzeichnet, daß man
- eine Probe aus der Mundhöhle entnimmt,
- nach der Probenentnahme eine zwischen zwei Primer-Sequenzen des genannten Bakteriums angeordnete Ziel-DNA amplifiziert nach dem PCR-Verfahren unter Verwendung der genannten Primer, wobei die Primer für den Nachweis der Ziel-DNA von Bacteroides gingivalis die folgenden Sequenzen haben:
Bg1 : 5'CTC TTG CTA TGA CAG CTT GT3'
Bg2 : 5'AGT CAG TTC AGT TGT CAA TG3' und
die Primer für den Nachweis der Ziel-DNA von Actinobacillus actinomycetemcomitans die folgenden Sequenzen haben:
Aa1: 5'GGG TCT GGA GTA CTT TAG GG3'
Aa2: 5'CCC AGG CGG TCA GAT TTA3'
- nach der Amplifikation die Amplifikations-Produkte der Ziel-DNA mit einer für die Ziel-DNA spezifischen Sonde hybridisiert und
- die Hybridisierung nachweist (anzeigt).

2. Verfahren nach Anspruch 1, dadurch gekennzeichnet, daß man für den Nachweis mehrerer Bakterien die Amplifikation in dem gleichen Behälter mit Primern durchführt, die mit den genannten Bakterien kompatibel (verträglich) sind.

3. Primer für den Nachweis der Ziel-DNA von Bacteroides gingivalis, welche die folgenden Sequenzen aufweist:
Bg1 : 5'CTC TTG CTA TGA CAG CTT GT3'
Bg2: 5'AGT CAG TTC AGT TGT CAA TG3',

4. Primer für den Nachweis der Ziel-DNA von Actinobacillus actinomycetemcomitans, welche die folgenden Sequenzen aufweisen:
Aa1 : 5'GGG TGT GGA GTA CTT TAG GG3'
Aa2: 5'CCC AGG CGG TCA GAT TTA3'.

5. Sonde, die für die Ziel-DNA von Bacteroides gingivalis spezifisch ist, dadurch gekennzeichnet, daß es sich dabei handelt um die Sonde:
Bg: GTT GAA GAC ATC AAA TGT AGT GCA GGC CAA.

6. Sonde, die für die Ziel-DNA von Actinobacillus actinomycetemcomitans spezifisch ist, dadurch gekennzeichnet, daß es sich dabei handelt um die Sonde
AaC18: CCC CAA ATC GGG ACC ACG.

7. Set zur Durchführung des Verfahrens nach einem der Ansprüche 1 oder 2.

8. Set nach Anspruch 7, dadurch gekennzeichnet, daß die Primer und/oder die Sonden diejenigen der Ansprüche 3 bis 6 sind.
